(19) Europäisches Patentamt

European Patent Office

Office européen des brevets

(11) Veröffentlichungsnummer: **0 312 073 B1**

(12) **EUROPÄISCHE PATENTSCHRIFT**

(45) Veröffentlichungstag der Patentschrift: **04.03.92**

(21) Anmeldenummer: **88117083.1**

(22) Anmeldetag: **14.10.88**

(51) Int. Cl.5: **A61M 39/00**

(54) **Ansatzstück mit Ventil.**

(30) Priorität: **15.10.87 DE 3734894**

(43) Veröffentlichungstag der Anmeldung:
**19.04.89 Patentblatt 89/16**

(45) Bekanntmachung des Hinweises auf die
Patenterteilung:
**04.03.92 Patentblatt 92/10**

(84) Benannte Vertragsstaaten:
**AT BE CH DE ES FR GB IT LI NL SE**

(56) Entgegenhaltungen:
**EP-A- 0 172 629**
**EP-A- 0 189 651**
**BE-A- 372 374**
**US-A- 2 211 759**

(73) Patentinhaber: **B. Braun Melsungen AG**
**Carl-Braun Strasse**
**W-3508 Melsungen(DE)**

(72) Erfinder: **Haindl, Hans-Günter**
**Schoene Aussicht 4**
**W-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Ansatzstück für ein langgestrecktes rohrförmiges Element, das in einen Körperhohlraum einführbar ist, insbesondere für ein Kunststoffkapillar, einen Katheterschlauch oder eine ähnliche medizinische Leitung, bestehend aus einem Gehäuse mit einem eine Einlaßöffnung und eine Auslaßöffnung verbindenden Kanal, der einen an der Innenwand des Gehäuses anliegenden flexiblen koaxialen Schlauchabschnitt enthält, wobei das Gehäuse zwei koaxiale Rohrteile aufweist, die im Bereich des Schlauchabschnittes über ein Gelenk knickbar miteinander verbunden sind.

Zum Punktieren von Blutgefäßen zur Blutabnahme, zur Infusion und zum Einführen von Venenkathetern werden sogenannte Venenverweilkanülen benutzt, die aus einer Stahlkanüle bestehen, welche in ein Kunststoffkapillar eingeschoben ist. An dem Kunststoffkapillar ist ein Ansatzstück befestigt, mit dem ein Ansatz der Stahlkanüle zusammengesteckt ist. Mit der über das Ende des Kunststoffkapillars vorstehenden angeschliffenen Spitze der Stahlkanüle wird die Haut punktiert und es werden Stahlkanüle und Kunststoffkapillar ein Stück in das Blutgefäß vorgeschoben. Danach wird die Stahlkanüle aus dem Kunststoffkapillar herausgezogen und es kann an das Ansatzstück ein Verbindungsteil eines Flüssigkeitüberleitungssystems angeschlossen bzw. ein Venenkatheter durch das Kunststoffkapillar in das Blutgefäß eingeschoben werden. Nach Herausziehen der Stahlkanüle strömt Blut in das Kunststoffkapillar ein und tritt aus dem Ansatzstück aus, was zur Infektion des Anwenders führen kann. Bei der Punktion zentraler Venen kann es bei negativem Venendruck auch zum Einschleppen von Luft in das Kunststoffkapillar und damit zur Luftembolie kommen. Um diese beiden für den Patienten schädlichen Einflüsse auszuschalten, sind Ansatzstücke mit Verschließvorrichtungen für ihren Durchlaß ausgerüstet worden. Beispielsweise ist ein Anschlußstück bekannt (DE 28 17 102 C2), in dessen Durchflußkanal eine Scheibe aus elastomerem Material eingesetzt ist, die eine zentrale Schlitzung aufweist, die von einer verschiebbaren Hülse öffenbar ist, welche mittels eines Spritzenkegels betätigt wird. Diese Ausbildung ist für eine Venenverweilkanüle ungünstig, weil nach längerer Lagerung des mit einem Kunststoffkapillar verbundenen Anschlußstückes auf der Stahlkanüle an den Schlitzlippen eine bleibende Eindrückung auftritt, die eine dichte Aneinanderlegung der Schlitzlippen zum Verschluß des Scheibendurchlasses verhindert.

Bei einem bekannten Ansatzstück der eingangs erwähnten Art (DE 29 41 278 C2) wird der flexible Schlauchabschnitt in dem Gehäuse zum Verschluß von mindestens einem Druckkörper in Form einer Kugel zusammengequetscht. Jene Kugel sitzt in einer Wandöffnung des Gehäuses und wird von einem auf der Außenfläche des Gehäuses axial beweglichen Schieber mit Angriffsflächen für die Kugeln radial einwärts gedrückt. Flache Rastaussparungen für einen Kugelkalottenbereich in den Angriffsflächen des Schiebers halten diesen in Schließstellung des Schlauchabschnittes fest. Durch dieses Einrasten des Schiebers muß bei jeder Umstellung ein Druckpunkt überwunden werden, der die Schieberbetätigung erschwert, insbesondere wenn dies mit derjenigen Hand erfolgen muß, die das Kunststoffkapillar oder den Katheterschlauch festhält. Dieses Ansatzstück mit Verschließvorrichtung ist infolge der zahlreichen Einzelbestandteile, die präzise zusammengebaut werden müssen, teuer.

US 38 51 647 betrifft ein Ansatzstück, dessen Gehäuse aus einem gummielastischen Schlauchkörper gebildet ist, der sich aus der Achslinie des Katheters seitwärts wegbiegen läßt, um den Weg zum Einschieben einer Punktionskanüle in den Katheter freizugeben. Zu diesem Zweck ist eine Umfangshälfte der Spitze des Schlauchkörpers mit der Hälfte eines starren Trichters verklebt, der mit dem Katheter fest verbunden ist. Die andere Umfangshälfte der Spitze läßt sich von der Punktionskanüle seitlich wegschieben, so daß sie ungehindert durch den Trichter in den Katheter einführbar ist. Um den Verschluß des das Katheterlumen fortsetzenden Kanals geht es bei diesem Ansatzstück nicht.

Ferner ist ein Schlauchverschluß bekannt (DE 83 21 458 U1), der eine Sollbruchstelle in einer Membran aufweist, die in Schließstellung das Lumen des Schlauches überspannt und durch energische Knickbewegung des Schlauches zertrennt wird. Ein Wiederverschluß ist unmöglich.

Ferner beschreibt BE-A-372374 einen Schlauchverschluß der ein einen Schlauch enthaltendes Gehäuse aufweist, wobei das Gehäuse aus zwei über ein Gelenk verbundenen, koaxialen Rohrteilen besteht.

Der Erfindung liegt die Aufgabe zugrunde, ein Ansatzstück, das einen flexiblen Schlauchabschnitt als Verschlußelement aufweist, so zu verbessern, daß es sich einfach handhaben und preiswert anfertigen läßt.

Diese Aufgabe wird bei einem Ansatzstück der eingangs genannten Art erfindungsgemäß dadurch gelöst, daß der Schlauch abschnitt abdichtend gegen die Innenwand des Gehäuses anliegt und daß einer der beiden Rohrteile an der Knickkehlenseite des Gehäuses einen axial gerichteten Fortsatz aufweist, der in Streckposition des Gehäuses in einen komplementären Ausschnitt des anderen Rohrteiles eingreift und in Knickposition des Gehäuses den Schlauchabschnitt bis zum Verschluß seines Durchlasses abquetscht.

Eine solche Verschließvorrichtung bei einem Ansatzstück läßt sich problemlos handhaben, indem zum Verschluß des Kanals des Gehäuses dieses einfach geknickt und dabei der Schlauchabschnitt so deformiert wird, daß die Innenfläche seiner Wand auf der Seite der Knickkehle gegen die Innenfläche seiner Wand auf der gegenüberliegenden Seite anstößt und gegen diese angedrückt wird. Der Aufbau ist einfach und preiswert herstellbar. Es können keine beweglichen Teile durch Einbau- oder Toleranzfehler bzw. durch anhaftendes Körperfluid verklemmen oder festkleben, so daß die Verschließvorrichtung zuverlässig einsatzbereit ist und eine ausreichende Abdichtung gegen das Ausströmen von Körperfluid aus dem Ansatzstück und das Einschleppen von Luft in das Ansatzstück vermittelt. Bei Anwendung des Ansatzstückes für das Kunststoffkapillar einer Venenverweilkanüle umgibt der Schlauchabschnitt die innenliegende Stahlkanüle spannungsfrei, so daß ihr elastisches Deformationsvermögen unabhängig von der Länge der Lagerung der Venenverweilkanüle zum Knickverschluß des Durchlasses voll verfügbar ist.

Die Abquetschung des Schlauchabschnittes ergibt sich bei einer Verschwenkung der beiden Rohrteile zueinander um einen Winkel von etwa 90°. Um eine Abquetschung des Schlauchschnittes bereits bei geringerer gegenseitiger Auslenkung der Rohrteile, d.h. bei einem Winkel von über 90° in der Knickkehle zwischen beiden Rohrteilen, zu erzielen, ist erfindungsgemäß vorgesehen, daß einer der beiden Rohrteile an der Knickkehlenseite des Gehäuses den axial gerichteten Fortsatz aufweist, der in Streckposition des Gehäuses in einen komplementären Ausschnitt des anderen Rohrteiles eingreift. Bei Verschwenkung des einen Rohrteiles in bezug auf den anderen Rohrteil wird das Ende des Fortsatzes gegen den Schlauchabschnitt gedrückt und durch axiale Auseinanderlegung der Gelenkstelle und des Angriffspunktes des Fortsatzes an dem Schlauchabschnitt kommt die Innenfläche der einen Wandseite des Schlauchabschnittes bereits dann an der Innenfläche der anderen Wandseite des Schlauchabschnittes zur Anlage, wenn der patientenferne Rohrteil noch nicht um 90°, sondern je nach Länge des axialen Fortsatzes z.B. erst um 45° in bezug auf den patientennahen Rohrteil verschwenkt wurde.

Da das Gehäuse zwei koaxiale Rohrteile aufweist, die über das Gelenk schwenkbar miteinander verbunden sind, trägt der patientenseitige Rohrteil, an den das Kunststoffkapillar einer Venenverweilkanüle angesetzt sein kann, vorzugsweise eine quergerichtete Griffplatte, die das Festhalten dieses Teiles des Gehäuses mit einer Hand ermöglicht, während die andere Hand nach der Punktion die Stahlkanüle bis in den patientenfernen Rohrteil aus dem Ansatzstück herauszieht und mit ihr den zweiten Rohrteil um das Gelenk schwenkt, um ihn von der Haut des Patienten abzuheben. Hierdurch werden die beiden Rohrteile unter einem Winkel zueinander angestellt und das Gehäuse erhält einen Knick, der sich auf den innenliegenden Schlauchabschnitt so auswirkt, daß dieser durch Eindrückung abgequetscht wird, bis sein Durchlaß verschlossen ist.

Wenn die Winkelposition der Rohrteile gesichert ist, kann die Stahlkanüle herausgezogen werden, ohne daß es zum Herauslecken von Blut oder zum Eintreten von Luft kommen kann. Nach Anschließen eines Infusions-Systems, bzw. eines Venenkathetereinführungsstückes wird der angehobene Rohrteil heruntergedrückt, so daß er wieder in seine Ausgangslage zurückgeht. Die Infusion kann jetzt ungehindert durch das gestreckte Gehäuse einlaufen, bzw. der Venenkatheter kann vorgeschoben werden. Bei nur geringfügiger Abwinkelung der beiden Rohrteile (großer Winkel an der Knickkehle) verbleibt ein verengter Durchlaß in dem Schlauchabschnitt, durch den z.B. mit der in dem patientenfernen Rohrteil steckenden Stahlkanüle oder dem Infusionssystem gedrosselt Medikamente in den Patienten eingeführt werden können.

Zur Ermöglichung einer Kontrolle der Position der Stahlkanülenspitze von außen ist das Gehäuse vorzugsweise aus glasklarem Kunststoff hergestellt.

Die beiden Rohrteile können separate Rohrstücke sein, die über das als Bolzenscharnier ausgebildete Gelenk miteinander verbunden sind. Alternativ können die separaten Rohrstücke durch einen flexibel biegbaren Materialstreifen zusammengehalten sein, der nach der Art eines Filmscharnieres eine knieartige Biegung des Gehäuses an der Trennstelle zwischen beiden Rohrstücken erlaubt.

Eine weitere Möglichkeit der Gehäuseausbildung besteht darin, daß ein Rohrkörper mit geschlossener Umfangswand aus flexiblem Material zur Bildung der beiden Rohrteile bis auf einen schmalen Steg der Umfangswand in Querrichtung eingeschnitten ist. In diesem Falle bildet der schmale flexible Steg der Umfangswand ein inkorporiertes Scharnier, das beide Rohrteile gelenkig miteinander verbindet.

Unabhängig von der Ausbildung des Gelenkes ist es zweckmäßig, daß die beiden Rohrteile in abgewinkelter Position zueinander arretierbar sind. Einige Ausführungsbeispiele der Sicherung des geknickten Gehäuses sind in den Zeichnungen veranschaulicht.

Das Ansatzstück eignet sich nicht nur für die Verbindung mit dem Kunststoffkapillar einer Venenverweilkanüle, sondern es ist unter anderem gleichermaßen vorteilhaft für Venenkatheter anwendbar, bei denen Lufteintritt von außen verhindert werden muß.

In der Zeichnung sind Ausführungsbeispiele der Erfindung schematisch dargestellt. Es zeigen:

Fig. 1 einen Längsschnitt eines Ansatzstückes für das Kunststoffkapillar einer Venenverweilkanüle in Streckposition des Gehäuses,

Fig. 2 das Ansatzstück nach Figur 1 in Knickposition des Gehäuses,

Fig. 3 eine Draufsicht auf das Ansatzstück gemäß Figur 1 in Richtung des Pfeiles A,

Fig. 4 einen Längsschnitt einer zweiten Ausführungsform eines Ansatzstückes in Streckposition des Gehäuses und

Fig. 5 das Ansatzstück nach Figur 4 in Knickposition des Gehäuses.

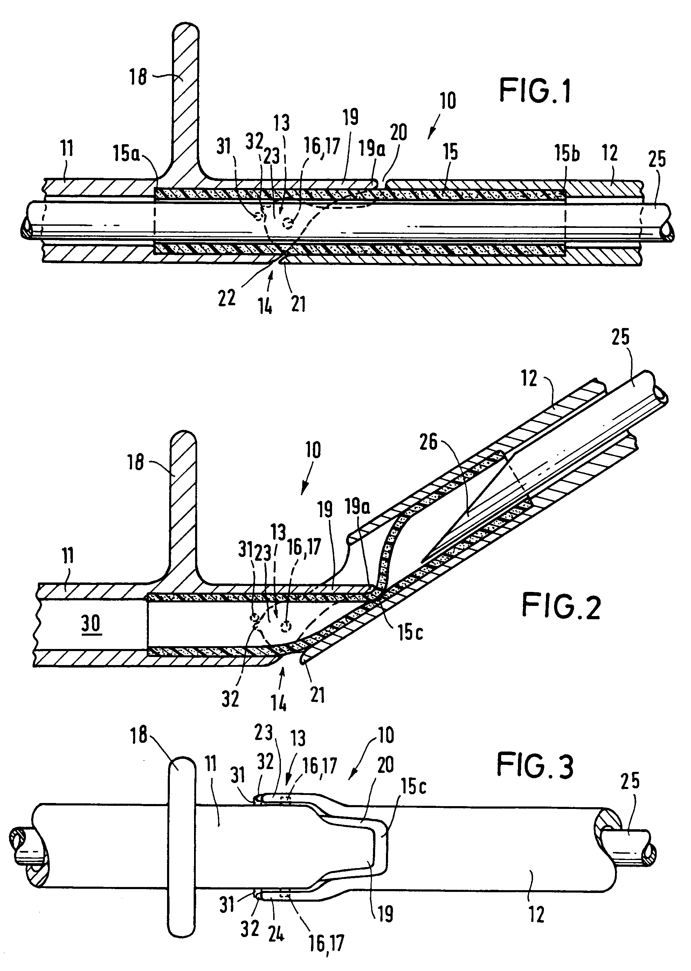

Ein Gehäuse 10 eines Ansatzstückes, das in den Figuren 1 und 2 zur Verbindung mit dem nicht gezeichneten Kunststoffkapillar einer Venenverweilkanüle bestimmt ist, besteht aus zwei Rohrteilen 11 und 12, die als separate Rohrstücke vorzugsweise aus glasklarem Kunststoff hergestellt sind. Die beiden Rohrteile 11 und 12 haben innen und außen kreiszylindrischen Querschnitt und gleiche Abmessungen; sie sind koaxial zueinander ausgerichtet und an ihren einander zugewandten Enden durch ein Bolzenscharnier 13 gelenkig miteinander verbunden. Das Bolzenscharnier 13 bildet ein Gelenk 14, das eine Knickung des Gehäuses 10 ermöglicht. In den beiden Rohrteilen 11, 12 befindet sich im Bereich des Gelenkes 14 ein Schlauchabschnitt 15 mit kreiszylindrischem Querschnitt aus weichem Silikon, dessen Länge so bemessen ist, daß die Gelenkstelle mit beträchtlichem axialen Überstand von dem Schlauchabschnitt 15 bedeckt ist. Der Schlauchabschnitt 15 ist in eine Ringaussparung in den beiden Rohrteilen 11, 12 lose gegen die Innenfläche der Wand anliegend eingefügt und die Stirnflächen seiner beiden Enden 15a und 15b stoßen zur axialen Verschiebungssicherung an Ringschultern der Ringaussparung an.

Zur Bildung des Bolzenscharniers 13 ist der patientenferne Rohrteil 12 an seinem dem patientennahen Rohrteil 11 zugewandten Ende erweitert und mit zwei parallelen Gabellaschen 23, 24 versehen, die axial gerichtet sind und das Ende des Rohrteiles 11 flankieren. Die Gabellaschen 23, 24 sind auf der Innenfläche abgerundeter Kopfenden mit auf einer gemeinsamen Quermittellinie liegenden Sacklöchern 16 ausgestattet, in die entsprechend angeordnete Nasen 17 an der Außenfläche des patientenfernen Rohrteiles 11 eingreifen. Der Rohrteil 11 weist an einer Seite der Knickkehle in der Nähe des Gelenkes 14 eine nach außen quergerichtete Griffplatte 18 auf. An der gleichen Seite ist der Rohrteil 11 über das Bolzenscharnier 13 hinaus durch einen axialen Fortsatz 19 verlängert, der aus der Umfangswand des Rohrteiles 11 gebildet ist. Der axiale Fortsatz 19 bildet eine Zunge mit stumpfem Ende, die bei Knickung des Gehäuses 10 von der Seite her durch einen komplementären Ausschnitt 20 am Ende des Rohrteiles 12 in dessen Kanal eindringen kann.

In der in Figur 1 gezeigten gestreckten Position des Gehäuses 10 ist der Schlauchabschnitt 15 unverformt und durch Anlage des dem Ausschnitt 20 und dem Fortsatz 19 gegenüberliegenden Randes 21 des Rohrteiles 12 gegen den Rand 22 des Rohrteiles 11 werden beide Rohrteile 11 und 12 in koaxialer Position gehalten, so daß eine Stahlkanüle 25 durch das Gehäuse 10 und ein an den patientennahen Rohrteil 11 angeschlossenes Kunststoffkapillar hindurchgesteckt sein kann, ohne daß der Schlauchabschnitt 15 in irgendeiner Weise deformiert oder gequetscht wird. Nach Punktion eines Blutgefäßes mit der Stahlkanüle 25 wird diese aus dem Ansatzstück 10 so weit herausgezogen, daß ihre angeschärfte Spitze 26 sich noch in dem Rohrteil 12 befindet (Fig.2) und das Bolzenscharnier 13 freigegeben ist. Mit Hilfe der Stahlkanüle 25 wird der Rohrteil 12 in bezug auf den auf der Haut des Patienten aufliegenden Rohrteil 11 verschwenkt. Die Knickung des Gehäuses 10 hat zur Folge, daß die eine Wandseite des Schlauchabschnittes 15 um die Stirnkante 19a des axialen Fortsatzes 19 des Rohrteiles 11 nach oben weggezogen und die gegenüberliegende Wandseite des Schlauchabschnittes 15 der an der Stirnkante 19a festgelegten Schlauchabschnittzone 15c genähert wird. Die Schwenkbewegung des Rohrteiles 12 ist dann beendet, wenn die Innenfläche der Schlauchabschnittzone 15c fest gegen die Innenfläche der gegenüberliegenden Wandseite des Schlauchabschnittes 15 gedrückt ist und der Kanal 30 durch Abquetschung des Schlauchabschnittes 15 abgesperrt wird.

Zur Arretierung dieser Knickposition des Gehäuses 10 dienen zwei auf der Außenseite des Rohrteiles 11 entgegengesetzt angeordnete Vorsprünge 31, die von Nasen 32 an den Enden der Gabellaschen 23, 24 des Rohrteiles 12 untergriffen werden (Fig. 2). Sobald die Nasen 32 über die Vorsprünge 31 hinweggeschnappt sind, kann die Stahlkanüle 25 aus dem Rohrteil 12 herausgezogen werden, ohne daß es zum Herauslecken von Blut oder zum Eintreten von Luft kommen kann. Nach Anschließen eines Infusionssystems bzw. eines Venenkatheter-Einführungsstückes an ein Verbindungselement an dem Rohrteil 12, wird dieser wieder heruntergedrückt, so daß er durch Hinüberschnappen der Nasen 32 über die Vorsprünge 31 wieder in seine Ausgangslage zurückgeht und das Gehäuse 10 einen geraden Rohrkörper bildet, der gegen die Haut des Patienten anliegt. Die Infusion kann jetzt ungehindert durch das gestreckte gerade Gehäuse 10 einlaufen bzw. es kann ein Venenkatheter vorgeschoben werden.

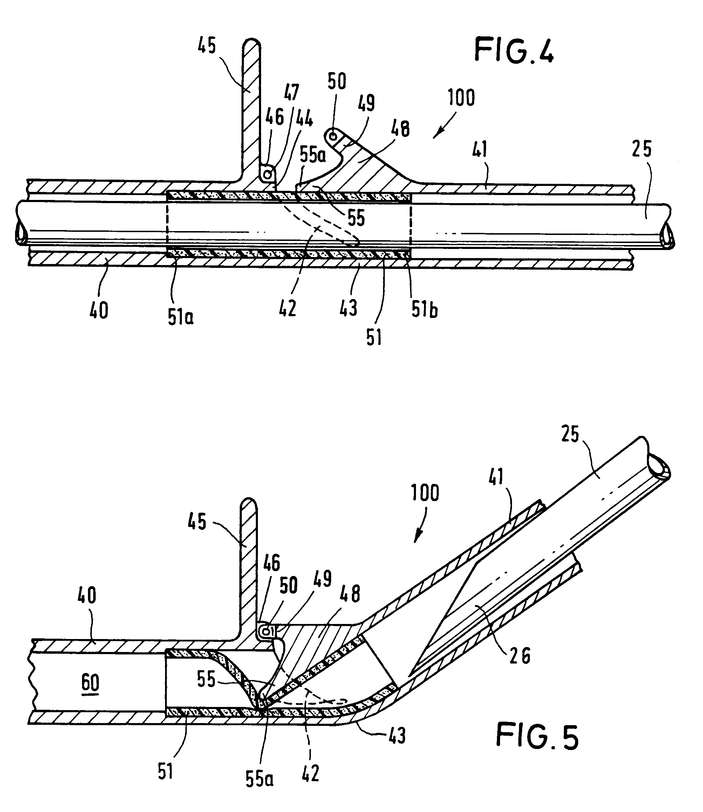

Bei dem Beispiel der Figuren 4 und 5 ist ein Gehäuse 100 eines Ansatzstückes als Spritzgußteil aus glasklarem flexiblem Kunststoffmaterial in Form eines Rohrkörpers hergestellt. Der Rohrkörper weist zur Bildung von zwei Rohrteilen 40, 41 einen schrägen Einschnitt 42 auf, der von dem patientennahen Rohrteil 40 zu dem patientenfernen Rohrteil 41 verläuft und mit Abstand zu der Umfangswand des Rohrkörpers endet. Auf diese Weise bleibt auf einer Wandseite ein schmaler Steg 43 der Umfangswand des Rohrkörpers stehen, der als Scharnier wirksam ist und eine gelenkige Verschwenkung des Rohrteiles 41 in bezug auf den Rohrteil 40 ermöglicht (Fig. 5). Das Scharnier bildet im Prinzip ein Filmscharnier; bei geeigneter Werkstoffwahl übernimmt also der Restquerschnitt des Rohrkörpers Scharnierfunktion. Auf der anderen Wandseite des Rohrkörpers entsteht ein axialer Fortsatz 55 des Rohrteiles 41.

Der Rohrteil 40 ist im Bereich des Einschnittes 42 mit einer quer nach außen abgehenden Griffplatte 45 ausgestattet. Am Fuß der Griffplatte 45 sind auf der dem Einschnitt 42 zugewandten Seite zwei kleine parallele Plättchen 46 ausgebildet, die mit einem Loch 47 versehen sind. An dem patientenfernen Rohrteil 41 des Rohrkörpers ist auf der Seite der Griffplatte 45 auf dem Fortsatz 55 eine nach außen ragende Dreiecksplatte 48 angeformt. Die Dreiecksplatte 48 trägt an ihrer Spitze einen geraden Arm 49, der in Richtung der Griffplatte 45 weist und an dessen Ende auf beiden Seitenflächen je ein Knopf 50 angeordnet ist.

In dem Rohrkörper liegt wandschlüssig ein Schlauchabschnitt 51 aus Silikon, dessen eines Ende 51a gegen eine Ringschulter in dem Rohrteil 40 anstößt und dessen anderes Ende 51b frei in den Rohrteil 41 hineinragt. Der Schlauchabschnitt 51 liegt lose in den beiden Rohrteilen 40, 41, d.h. er ist mit der Innenfläche der Rohrteilwandung nicht verbunden. In dem gestreckten Gehäuse 100 gemäß Figur 4 steckt eine Stahlkanüle 25, die den Streckzustand des Gehäuses 100 fixiert. Die angeschliffene Spitze der Stahlkanüle 25 steht über die Spitze eines Kunststoffkapillars vor, das an den Rohrteil 40 angeschlossen ist und nach Zurückziehen der Stahlkanüle 25 aus dem Gehäuse 100 in einem punktierten Blutgefäß verbleibt, um einen Einlaß für medizinische Applikationen zu vermitteln. Nach der Punktion eines Blutgefäßes wird mit Hilfe der in den Rohrteil 41 zurückgezogenen Stahlkanüle 25 der Rohrteil 41 hochgebogen, bis die beiden Knöpfe 50 an dem Arm 49 der Dreiecksplatte 48 in die Löcher 47 der beiden Plättchen 46 an dem Rohrteil 40 einrasten. Die Knickung des Gehäuses 100 erfolgt um das Materialgelenk 43 und das Ende 55a des Fortsatzes 55 drückt die gegen ihn anliegende Wandseite des Schlauchabschnittes 51 von der Innenfläche des Rohrteiles 40 nach innen weg gegen die Innenfläche der gegenüberliegenden Wandseite des Schlauchabschnittes 51. Bei arretierten Rohrteilen 40, 41 ist der Schlauchabschnitt 51 so verformt, daß der Kanal 60 des Gehäuses 100 abgesperrt ist und keine Flüssigkeit nach außen bzw. keine Luft nach innen dringen kann. Die übrige Handhabung dieses Ansatzstückes 100 entspricht der im Zusammenhang mit dem Beispiel der Figuren 1 bis 3 geschilderten Manipulation.

Der bei beiden Beispielen vorhandene axiale Fortsatz 19 bzw. 55 ermöglicht eine Zusammenquetschung des Schlauchabschnittes 15 bzw. 51 bereits dann, wenn der Rohrteil 12; 41 unter einem Winkel von nur etwa 45° zu dem waagerechten Rohrteil 11; 40 verschwenkt ist. Bei Fehlen axialer Fortsätze wäre eine 90° Verschwenkung der Rohrteile in bezug aufeinander erforderlich, um den Kanal 30 bzw. 60 durch Abquetschen des Schlauchabschnittes 15 bzw. 51 zu verschließen. Der Fortsatz kann alternativ bei beiden Beispielen an dem jeweils anderen Rohrteil ausgebildet sein.

Sofern es sich als zweckmäßig erweist, kann die gleiche Anordnung auch um 180° gedreht vorgesehen werden.

**Patentansprüche**

1. Ansatzstück für ein langgestrecktes rohrförmiges Element, das in einen Körperhohlraum einführbar ist, insbesondere für ein Kunststoffkapillar, einen Katheterschlauch oder eine ähnliche medizinische Leitung, bestehend aus einem Gehäuse (10;100) mit einem eine Einlaßöffnung und eine Auslaßöffnung verbindenden Kanal (30; 60), der einen an der Innenwand des Gehäuses (10; 100) anliegenden flexiblen koaxialen Schlauchabschnitt (15; 51) enthält, wobei das Gehäuse (10; 100) zwei koaxiale Rohrteile (11, 12; 40, 41) aufweist, die im Bereich des Schlauchabschnittes (15; 51) über ein Gelenk (14; 43) knickbar miteinander verbunden sind, **dadurch gekennzeichnet,** daß der Schlauchabschnitt (15; 51) abdichtend gegen die Innenwand des Gehäuses anliegt, und daß
einer der beiden Rohrteile (11, 12; 40, 41) an der Knickkehlenseite des Gehäuses (10, 100) einen axial gerichteten Fortsatz (19; 55) aufweist, der in Streckposition des Gehäuses (10; 100) in einen komplementären Ausschnitt des anderen Rohrteiles (12; 40) eingreift und in Knickposition des Gehäuses (10; 100) den Schlauchabschnitt (15; 51) bis zum Verschluß seines Durchlasses abquetscht.

2. Ansatzstück nach Anspruch 1,

**dadurch gekennzeichnet,** daß die beiden Rohrteile (11, 12) separate Rohrstücke sind und daß das Gelenk (14) als Bolzenscharnier (13) ausgebildet ist.

3. Ansatzstück nach Anspruch 1,
   **dadurch gekennzeichnet,** daß die beiden Rohrteile (11, 12) separate Rohrstücke sind und daß das Gelenk (14) als Filmscharnier aus flexibel biegbaren Materialstreifen gebildet ist.

4. Ansatzstück nach Anspruch 1,
   **dadurch gekennzeichnet,** daß ein Rohrkörper mit geschlossener Umfangswand aus flexiblem Material zur Bildung der beiden Rohrteile (40, 41) bis auf einen schmalen Steg (43) der Umfangswand in Querrichtung eingeschnitten ist.

5. Ansatzstück nach Anspruch 4,
   **dadurch gekennzeichnet,** daß der Einschnitt (42) zur Längsachse des Rohrkörpers schräg verläuft.

6. Ansatzstück nach einem der Ansprüche 1 bis 5,
   **dadurch gekennzeichnet,** daß die beiden Rohrteile (11, 12; 40, 41) in Knickposition des Gehäuses (10; 100) zueinander arretierbar sind.

7. Ansatzstück nach einem der Ansprüche 1 bis 6,
   **dadurch gekennzeichnet,** daß das Gehäuse (10; 100) aus glasklarem Kunststoff hergestellt ist.

## Claims

1. A joining member for an elongate tubular element introducible into a body cavity, in particular for a plastics capillary, a catheter tube or a similar medical conduit, consisting of a housing (10; 100) with a channel (30; 60) connecting an inlet opening and an outlet opening, said channel holding a flexible co-axial tube portion (15; 51) abutting the inner wall of said housing (10; 100), said housing (10; 100) having two coaxial pipe portions (11, 12; 40, 41) that are kinkably connected in the area of said tube portion (15; 51) through a joint (14; 43),

   characterized in that said tube portion (15; 51) sealingly abuts said inner wall of said housing and that one of said two pipe portions (11, 12; 40, 41) is provided with an axially directed projection at the kink fillet side of said housing, which, in the straightened position of said housing (10; 100), engages into a complementary cut-out of the other pipe portion (12; 40) and, in the kinked position of said housing (10; 100), squeezes said tube portion until its passage is closed.

2. The joining member of claim 1, characterized in that said two pipe portions (11, 12) are separate pipe members and that said joint (14) is a pivot pin hinge (13).

3. The joining member of claim 1, characterized in that said two pipe portions (11, 12) are separate pipe members and that said joint (14) is a film hinge of flexibly bendable strips of material.

4. The joining member of claim 1, characterized in that, in order to form said two pipe portions (40, 41), a pipe body with a closed circumferential wall of flexible material is cut in transversally, leaving a narrow ridge (43) of the circumferential wall.

5. The joining member of claim 4, characterized in that said cut (42) extends obliquely to the longitudinal axis of said pipe body.

6. The joining member of one of claims 1 to 5, characterized in that said two pipe portions (11, 12; 40, 41) may be arrested with respect to each other in the kinked position of said housing (10; 100).

7. The joining member of one of claims 1 to 6, characterized in that said housing (10; 100) is made of clear plastics material.

## Revendications

1. Embout pour un élément tubulaire allongé, qui peut être introduit dans une cavité d'un corps, notamment pour un capillaire en matière synthétique, un tuyau souple cathéter ou un conduit médical semblable, constitué par un boîtier (10;100) possédant un canal (30;60), qui raccorde une ouverture d'admission et une ouverture de sortie et contient une section coaxiale flexible de tuyau souple (15;51), adjacente à la paroi intérieure du boîtier (10;100), et dans lequel le boîtier (10;100) présente deux parties tubulaires coaxiales (11,12;40,41), qui sont reliées entre elles, de manière à pouvoir être fléchies, au moyen d'une articulation (14;43) dans la zone de la section (15;51) du tuyau souple, caractérisé en ce que la section de tuyau souple (15;51) s'applique d'une manière étanche contre la paroi intérieure du boîtier, et que l'une des deux parties tubulaires (11,12;40,41) présente, au côté évidé de fléchissement du boîtier (10,100), un prolongement axial (19;55), qui, lorsque le boîtier (10;100) est dans sa position étendue, s'engage dans une section complémentaire de l'autre partie tubulaire (12;40) et qui, lorsque le boîtier (10;100) est en position fléchie, comprime la section de tuyau souple (15;51) jusqu'à en fermer le passage intérieur.

2. Embout selon la revendication 1, caractérisé en ce que les deux parties tubulaires (11,12) sont des pièces tubulaires séparées, et que l'articulation (14) est réalisée sous la forme d'une charnière à tourillon (13).

3. Embout selon la revendication 1, caractérisé en ce que les deux parties tubulaires (11,12) sont des pièces tubulaires séparées et que l'articulation (14) est réalisée sous la forme d'une charnière en forme de pellicule formée de bandes de matériau flexibles et pliables.

4. Embout selon la revendication 1, caractérisé en ce qu'un corps tubulaire possédant une paroi circonférentielle fermée et constitué par un matériau flexible servant à former les deux parties tubulaires (40,41) est découpée dans la direction transversale hormis une petite barrette (43) de la paroi circonférentielle.

5. Embout selon la revendication 4, caractérisé en ce que la découpe (42) s'étend obliquement par rapport à l'axe longitudinal du corps tubulaire.

6. Embout selon l'une des revendications 1 à 5, caractérisé en ce que les deux parties tubulaires (11,12;40,41) peuvent être bloquées l'une par rapport à l'autre lorsque le boîtier (10;100) est dans la position fléchie.

7. Embout selon l'une des revendications 1 à 6, caractérisé en ce que le boîtier (10;100) est réalisé en une matière synthétique transparente.

FIG.1

FIG.2

FIG.3

FIG.4

FIG.5